Europäisches Patentamt

(19) European Patent Office

Office européen des brevets

(11) Publication number: **0 328 163 B1**

(12) **EUROPEAN PATENT SPECIFICATION**

(45) Date of publication of patent specification :
18.12.91 Bulletin 91/51

(51) Int. Cl.⁵: **A61M 5/14**

(21) Application number: **89105437.1**

(22) Date of filing : **10.04.84**

(54) **Fault detection apparatus for parenteral infusion system and method of detecting faults in such a system.**

(30) Priority : **11.04.83 US 483903**

(43) Date of publication of application :
**16.08.89 Bulletin 89/33**

(45) Publication of the grant of the patent :
**18.12.91 Bulletin 91/51**

(84) Designated Contracting States :
**CH DE FR GB LI**

(56) References cited :
**EP-A- 0 096 849**
**FR-A- 2 091 101**
**US-A- 3 690 318**
**US-A- 4 267 834**
**US-A- 4 392 847**

(60) Publication number of the earlier application in
accordance with Art. 76 EPC : **0 121 931**

(73) Proprietor : **IVAC CORPORATION**
**10300 Campus Pt. Drive**
**San Diego, CA 92121 (US)**

(72) Inventor : **Nelson, Peter E.,Stanford College of**
**Medicine**
**Dept.of Anesthesia**
**Palo Alto,California 94305 (US)**

(74) Representative : **Howden, Christopher Andrew**
**et al**
**FORRESTER & BOEHMERT**
**Widenmayerstrasse 4/I**
**W-8000 München 22 (DE)**

Jouve, 18, rue Saint-Denis, 75001 PARIS

## Description

This invention relates generally to systems for administering parenteral fluids to a patient, and, more particularly, to systems of this type having an infusion apparatus for infusing the fluid into the patient's vascular system.

Systems of this particular type have enjoyed widespread usage in hospitals for administering parenteral fluids at precise rates. The systems are useful for both venous and arterial infusions and typically include an infusion pump and an associated controlling device for pumping the parenteral fluid through a fluid tube and needle to the patient's vein or artery.

One drawback to conventional infusion pump systems of this type is that the needle can sometimes become dislodged from the patient's vein or artery. This will normally cause an increase in back pressure, but the pump will nevertheless continue to pump fluid at substantially the same fixed rate. The fluid therefore can infiltrate into the patient's body tissue and cause severe damage. Similarly, the needle can sometimes become dislodged from the patient entirely, yet the pump will continue to pump the fluid at the same fixed rate.

One known prior technique for detecting fluid infiltrations is to monitor the patient's skin temperature in the vicinity of the needle. Since the parenteral fluid is ordinarily cooler than the patient's body temperature, and since the fluid is not carried away as rapidly when an infiltration occurs, an infiltration will ordinarily create a temperature drop in the vicinity of the needle. Thus, whenever a drop in skin temperature is detected, it is deduced that an infiltration is occurring. This technique is not believed to have proven completely satisfactorily in all circumstances, such as, for example, when the parenteral fluid has a temperature substantially the same as that of the patient's blood.

Other known prior techniques for detecting an infiltration of a parenteral fluid into a patient's body tissue involve intervention by hospital personnel. In one such technique, an attendant visually inspects the region around the needle, to detect any swelling that might indicate an infiltration. In another technique, useful only when the fluid is being administered from a bottle under the force of gravity, the attendant periodically lowers the bottle to an elevation below the needle such that fluid flows outwardly from the patient. If when this is done the patient's blood does not appear in the fluid tube, it can be deduced that the needle is not in fluid communication with a vein or artery. Neither of these techniques has proven to be entirely satisfactory, one reason being that they both require the presence of trained hospital personnel and cannot be performed automatically.

Still another prior technique for detecting infiltrations and other fault conditions is used in a parenteral administration system that regulates flow rate using a pinch valve located in the fluid tube, between a drop chamber and the patient. In particular, the pinch valve is controllably adjusted in order to maintain the frequency of fluid drops into the drop chamber at a selected value. If the limits of the pinch valve are exceeded in attempting to maintain the selected drop frequency, it is deduced that a fault condition is present. Operator intervention is still required, however, in order to determine the particular type of fault condition, e.g. an infiltration, that is present.

EP-0096849-A3, published 28th December 1983, discloses a method and apparatus for detecting occlusions in a parental administration system of a type having a peristaltic pump for pumping a fluid through a feeding tube to a patient. A pressure transducer measures the cyclicly varying pressure of the fluid in the feeding tube and produces a corresponding pressure signal, and a comparator compares the pressure signal to a selected one of several thresholds, depending on the current stage of the pumping cycle. An occlusion alarm is actuated whenever the selected threshold is exceeded. The device of EP 0096849 A3 is still, however, limited in its applications.

DE-A-3018641 discloses a parenteral infusion system having a peristaltic pump for infusing fluid into a vein of a patient. A pressure operated switch is operated when the pressure of the fluid supplied to the patient exceeds a predetermined level. Similarly, a flow sensor senses the rate of flow of infusion fluid to the patient. The apparatus operates an alarm and stops the peristaltic pump when the pressure switch is not operated and the flow rate is zero ; when the flow rate is zero and the pressure switch is operated or when the pressure switch is not operated but the flow rate is high. In the apparatus of DE-A-3018641, however, the pressure operated switch is simply a two-state device being in a first state when the pressure is below a predetermined threshold and in a second state when the pressure is above the predetermined threshold.

US-A-4129125 discloses a heartbeat see e.g. also US-A-2699465 sensing device wherein microphones or electrodes are employed to sense the heart activity. A microprocessor "filters out" ambient pulses which are the result of muscle movements etc, (where electrodes are used) or filters out ambient noises (where microphones are used). Although US-A-4129125 contains little information as to the filtering involved, it seems highly probable that the "filtering" concerned is, at least to the extent that it is frequency selective, low-pass filtering, although it may be that the "filtering" in question, since it is carried out by the microprocessor, is a synchronisation technique of the kind in which a detection "window" is opened cyclically and signals are detected as heartbeat signals only if they occur within the window concerned. This technique is, of course, least effective in eliminating signals of long duration, (i.e. low frequency signals) and most effective in filtering out signals of short duration (i.e. having substantial high frequency components). It further seems, since the "filtering" is said to be carried out by the microprocessor, that there will be no filtering, in a strict sense, in the device of US-A-4129125, i.e. in the sense of there being an identifiable "signal" after "filtering" which corresponds with a signal before filtering with some components removed.

US-A-4277227 and US-A-4267834, which forms the basis for the precharacterising part of claim 1, discloses apparatus which monitors the pressure of intravenous fluid supplied to a patient and acts to terminate such supply, by stopping the infusion pump, when a predetermined threshold is exceeded. Once again, the device is a simple two-state mechanism, being in one state when the pressure is above a preset threshold and another state when the pressure is below that threshold.

It should be appreciated from the foregoing that there still is a need for an effective method and apparatus for automatically detecting faults such as infiltrations or an open line in a parenteral administration system of the type having an infusion device. The present invention fulfils this need.

According to one aspect of the present invention, there is provided a fault detection apparatus for use with a parenteral fluid administration system having an infusion device for infusing a parenteral fluid through a fluid tube into the vascular system of a patient, the apparatus including pressure transducer means for monitoring the pressure of the fluid in the fluid tube and producing a corresponding pressure signal ; characterised by means for high-pass filtering the pressure signal to produce an a.c. pressure signal, and means for evaluating the a.c. pressure signal to detect a dropout in signal components attributable to the patient's heartbeats and for producing the alarm signal if it detects such a dropout.

According to another aspect of the present invention, there is provided a method of detecting faults in operation of a parenteral fluid administration system having an infusion device for infusing parenteral fluid through a fluid tube to the various system of a patient, comprising the steps of monitoring the pressure of the fluid in the fluid tube and producing a corresponding pressure signal, characterised by the further steps of high-pass filtering the pressure signal to produce an a.c. pressure signal, evaluating the a.c. pressure signal to detect a dropout in signal components attributable to the patient's heartbeats and producing an alarm if such a dropout is detected.

An embodiment of the present invention is described below by way of example with reference to the accompanying drawings in which :

FIGURE 1 is a block diagram of a parenteral administration system having circuitry for detecting venous infiltrations, arterial infiltrations and open fluid lines and ;

FIGURE 2 is a simplified schematic diagram of an arterial infiltration detector suitable for use in the system of Figure 1.

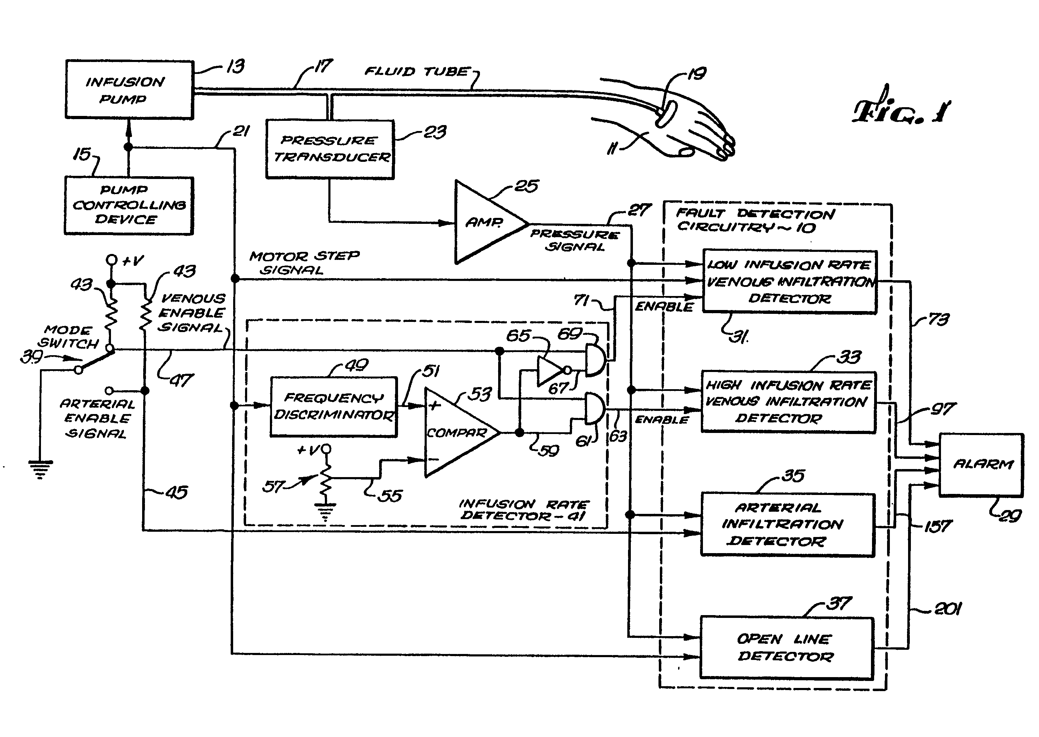

Referring now to the drawings, and particularly to Figure 1, there is shown fault detection circuitry 10 for use in a system for administering a parenteral fluid to the vascular system of a patient 11. The system includes a conventional infusion pump 13 and associated pump controlling device 15 for pumping the parenteral fluid through a fluid tube 17 and needle 19 to the patient. The pump is preferably of the peristaltic type, which pumps the fluid in a cyclic fashion. One such suitable pump and an associated controller for controlling its speed are described in a copending and commonly-assigned application for U.S. Patent Serial No. 06/281848 filed July 9, 1981, in the names of Stephen H. O'Leary et al and entitled "Method and Apparatus for Fluid Flow Control".

The pump controlling device 15 outputs a motor step signal for coupling on line 21 to the infusion pump 13. The signal is a sequence of pulses, each of which increments the pump by one step, to infuse a predetermined volume of parenteral fluid to the patient 11.

The parenteral administration system further includes a pressure transducer 23 and an associated amplifier 25. for monitoring the fluid pressure in the fluid tube 17 and producing a corresponding pressure signal for output on line 27.

The fault detection circuitry 10 evaluates the pressure signal on line 27 to detect certain characteristic patterns indicative of an improper fluid communication between the fluid tube 17 and the patient's vascular system. An alarm 29 is actuated if the circuitry detects such a condition. Such fault conditions include infiltrations of the fluid into body tissue other than the patient's vascular system, as well as a complete dislodging of the needle 19 from the patient 11 or a leak or air bubble in the fluid tube. In this way, a proper administration of the parenteral fluid can be ensured without the need for frequent monitoring or testing by hospital personnel.

More particularly, the fault detection circuitry 10 includes a low infusion rate venous infiltration detector 31 for detecting infiltrations when the infusion pump 13 is pumping parenteral fluid into the patient's venous system at a relatively low rate, and a high infusion rate venous infiltration detector 33 for detecting infiltrations when the pump is pumping fluid into the venous system at a relatively high rate. The fault detection circuitry further includes an arterial infiltration detector 35 for detecting infiltrations when the pump is pumping fluid into the patient's arterial system, and an open line detector 37 for detecting when there is a leak of some kind or an air bubble in the fluid connection between the pump and the patient 11.

The system further includes a mode switch 39 for indicating whether the system is intended to administer parenteral fluid to the patient's venous system or arterial system. The system also includes an infusion rate detector circuit 41, operable whenever the switch indicates that the system is pumping fluid into the patient's venous system to indicate whether the fluid is being pumped at a relatively high rate or a relatively low rate. The switch 39 and detector circuit 41 are used to enable operation of the appropriate venous infiltration detector circuit 31 or 33 or arterial infiltration detector circuit 35, depending on the system's operating mode.

More particularly, the mode switch 39 is a single-pole, double-throw switch having its middle terminal connected directly to ground and its two remaining terminals connected through separate resistors 43 to a positive voltage. The binary signals present on these two terminals are therefore opposite in phase to each other. One such signal is defined to be an arterial enable signal and the other is defined to be a venous enable signal.

The arterial enable signal is coupled on line 45 directly to the arterial infiltration detector circuit 35, and the venous enable signal is coupled on line 47 to the infusion rate detector 41. The infusion rate detector circuit relays the venous enable signal to either the low infusion rate venous infiltration detector 31 or the high infusion rate venous infiltration detector 33, depending on the infusion rate being effected by the infusion pump 13.

The infusion rate detector circuit 41 includes a frequency discriminator 49 for monitoring the motor step signal present on line 21 and producing an output signal having a voltage level generally proportional to the motor step signal's frequency. This output signal is coupled on line 51 to the positive input terminal of a comparator 53, which compares it with a selected reference level coupled to its negative input terminal. The reference level is supplied on line 55 from the wiper of a potentiometer 57, whose remaining two terminals are connected between ground and a positive supply voltage. If the discriminator output signal exceeds the threshold, indicating that the infusion pump 13 is pumping at a relatively high rate (e.g. above about 40 ml per hour), the comparator outputs a positive voltage level. On the other hand, if the discriminator output signal does not exceed the threshold, indicating that the pump is pumping at a relatively low rate, the comparator outputs a low voltage level signal.

The signal output by the comparator 53 is coupled on line 59 to a first AND gate 61 for ANDing with the venous enable signal supplied on line 47 from the mode switch 39. This produces a high infusion rate venous enable signal for coupling on line 63 to the high infusion rate venous infiltration detector 33. The detector 33 is thereby enabled to detect infiltrations whenever a venous infusion is selected by the mode switch and the infusion rate exceeds the prescribed threshold.

The signal output by the comparator 53 of the infusion rate detector 41 is also coupled on line 59 to a NOT gate 65, for inversion and coupling in turn on line 67 to a second AND gate 69, where it is ANDed with the same venous enable signal present on line 47. The resulting low infusion rate venous enable signal is coupled on line 71 to the low infusion rate venous infiltration detector 31. This detector 31 is thereby enabled whenever a venous infusion is selected by the mode switch 39 and the infusion rate does not exceed the prescribed threshold.

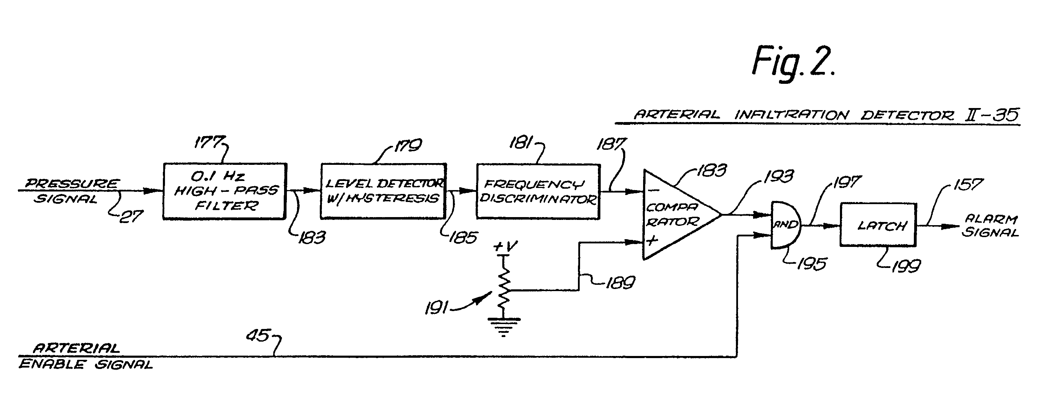

With reference now to Figure 2, there is shown a simplified schematic diagram of the arterial infiltration detector 35 of Figure 1. In the detector 35 infiltrations into the patient's body tissue separate from his arterial system are detected by monitoring the pressure signal to detect dropouts in the pressure variations caused by the patient's heartbeat.

More particularly, the arterial infiltration detector 35 of Figure 2 includes a 0.1 Hz high-pass filter 177, a level detector with hysteresis 179, a frequency discriminator 181 and a comparator 183. The high-pass filter filters the pressure signal supplied on line 27 to remove its dc level and pass only the successive pulses representative of the patient's heartbeats. The heartbeat pulses are present in the pressure signal whenever the fluid tube 17 and the needle 19 (Figure 1) are coupled directly to the patient's arterial system. The high-pass filtered signal is coupled on line 185 to the level detector, which converts the signal to a corresponding pulse sequence signal. The level detector detects only pulses having a magnitude of at least about 15 cm $H_2O$. The pulse sequence produced by the level detector is coupled on line 185 to the frequency discriminator, which produces an output signal having a level proportional to the frequency of its input signal.

Thus, if the needle 19 is properly inserted into the patient's arterial system, the heartbeats will be represented in the pressure signal and the frequency discriminator 181 will output a relatively high voltage level signal. On the other hand, if the needle is not properly inserted into the patient's arterial system, the heartbeats will not be represented in the pressure signal and the frequency discriminator will output a relatively low level signal.

The discriminator output signal is coupled on line 187 to the negative input terminal of the comparator 183, which compared it with a selected positive reference level supplied to its positive input terminal on line 189 from a potentiometer 191. Thus, if the frequency discriminator output signal ever drops below the reference level, the comparator outputs a positive voltage level. The reference level preferably corresponds to a pulse frequency of about 15 beats per minute.

The signal output by the comparator 183 is coupled on line 193 to an AND gate 195, where it is ANDed with the arterial enable signal supplied on line 45. If both signals are at a positive level, it is deduced that an arterial infiltration has occurred and the AND gate outputs a corresponding signal for coupling on line 197 to a latch 199. The latch, in turn, outputs the alarm signal for coupling on line 157 to the alarm 29 (Figure 1).

The alarm 29 is responsive to any of the four alarm signals coupled to it on lines 73, 97, 157 and 201 from the fault detection circuitry 10. It includes a four-input OR gate that ORs together the four signals and actuates a visual or audible indicator whenever any of the signals is high. It further includes a switch for use in selectively clearing the latches or flip-flops at the output stage of each detector in the fault detection circuitry.

It should be appreciated from the foregoing description that the present invention provides an improved apparatus and related method for detecting fault conditions such as an infiltration or an open line in a parenteral administration system. The apparatus is particularly adapted for use with a system of the type that includes a pulsing infusion pump for incrementally pumping a parenteral fluid through a fluid tube and needle to a patient's vascular system.

## Claims

1. Fault detection apparatus for use with a parenteral fluid administration system having an infusion device (13) for infusing a parenteral fluid through a fluid tube (17) into the vascular system of a patient, the apparatus including pressure transducer means (23) for monitoring the pressure of the fluid in the fluid tube and producing a corresponding pressure signal; characterised by means (177) for high-pass filtering the pressure signal to produce an a.c. pressure signal, and means (179, 181, 183, 195, 199) for evaluating the a.c. pressure signal to detect a dropout in signal components attributable to the patient's heartbeats and for producing the alarm signal if it detects such a dropout.

2. A method of detecting faults in operation of a parenteral fluid administration system having an infusion device (13) for infusing parenteral fluid through a fluid tube (17) to the vascular system of a patient, comprising the steps of monitoring the pressure of the fluid in the fluid tube and producing a corresponding pressure signal, characterised by the further steps of high-pass filtering the pressure signal to produce an a.c. pressure signal, evaluating the a.c. pressure signal to detect a dropout in signal components attributable to the patient's heartbeats and producing an alarm if such a dropout is detected.

3. Apparatus according to claim 1 wherein said means for evaluating the a.c. pressure signal includes a level detector (179), with hysteresis, arranged to receive the filtered signal from said high-pass filter (177) and to convert said signal into a pulse sequence signal, a frequency discriminator (181) arranged to receive said pulse sequence signal and to produce an output signal having a level proportional to the frequency of said pulse sequence signal, and a comparator (183) having one input connected with the output of said frequency discriminator and another input connected with a voltage reference (191).

## Patentansprüche

1. Fehlererfassungsvorrichtung zur Verwendung mit einem Verabreichungssystem für parenterale Fluide, mit einer Infusionsvorrichtung (13) zum Einleiten eines parenteralen Fluides durch ein Fluidrohr (17) in das Gefäßsystem eines Patienten, wobei die Vorrichtung eine Druckwandlereinrichtung (23) zum Überwachen des Druckes des Fluides in dem Fluidrohr zum Erzeugen eines entsprechenden Drucksignales umfaßt ; gekennzeichnet durch Mittel (177) für die Hochpaßfilterung des Drucksignales zum Erzeugen eines Gleichstrom-Drucksignales und ein Mittel (179, 181, 183, 195, 199) zum Auswerten des Gleichstrom-Drucksignales, um einen Aussetzfehler in Signalkomponenten zu erfassen, welche den Herzschlägen des Patienten zuordenbar sind, und zum Erzeugen eines Alarmsignales, wenn es einen solchen Aussetzfehler erfaßt.

2. Verfahren zum Erfassen von Fehlern beim Betrieb eines Verabreichungssystemes für parenterales Fluid mit einer Infusionsvorrichtung (13) zum Einleiten parenteralen Fluids durch ein Fluidrohr (17) an das Gefäßsystem eines Patienten, mit den Schritten des Überwachens des Druckes des Fluids in dem Fluidrohr und des Erzeugens eines entsprechenden Drucksignales, gekennzeichnet durch die weiteren Schritte des Hochpaßfilterns des Drucksignales zum Erzeugen eines Gleichstrom-Drucksignales, Auswerten des Gleichstrom-Drucksignales zum Erfassen eines Aussetzfehlers in Signalkomponenten, welche dem Herzschlag des Patienten zuordenbar sind, und Erzeugen eines Alarms, wenn ein solcher Aussetzfehler erfaßt wird.

3. Vorrichtung nach Anspruch 1, bei der das Mittel zum Auswerten des Gleichstrom-Drucksignales einen Niveaudetektor (179), mit Hysterese, angeordnet zum Empfangen des gefilterten Signales aus dem Hochpaßfilter (177) und zum Umwandeln des Signales in ein Pulssequenzsignal, einen Frequenzdiskriminator (181), angeordnet zum Empfangen des Pulssequenzsignales und zum Erzeugen eines Ausgabesignals mit einem Wert proportional zur Frequenz des Pulssequenzsignales, und einem Komparator (183) mit einem Eingang, der mit dem Ausgang des Frequenzdiskriminators verbunden ist, und einem anderen Eingang, der mit einer Spannungsreferenz (191) verbunden ist, umfaßt.

## Revendications

1. Appareil de détection de défauts destiné à l'utilisation avec un système d'administration de fluide parentéral ayant un dispositif de perfusion (13) destiné à perfuser un fluide parentéral par l'intermédiaire d'un tube de fluide (17) dans le système vasculaire d'un patient, l'appareil comprenant un capteur de pression (23) pour surveiller la pression du fluide dans le tube de fluide et produire un signal de pression correspondant ; caractérisé par des moyens (177) destinés à filtrer en passe-haut le signal de pression pour produire un signal de pression en courant alternatif, et des moyens (179, 181, 183, 195, 199) destinés à évaluer le signal de pression en courant alternatif pour détecter une perte de niveau ou effacement dans les composantes du signal imputable à la fréquence cardiaque du patient et pour produire le signal d'alarme si une chute ou perte de niveau est détectée.

2. Procédé de détection de défauts dans le fonctionnement d'un système d'administration de fluide parentéral ayant un dispositif de perfusion (13) pour la perfusion de fluide parentéral par un tube de fluide (17) au système vasculaire d'un patient comprenant les étapes consistant à surveiller la pression du fluide dans le tube de fluide et à produire un signal de pression correspondant, caractérisé par les étapes supplémentaires de filtrage passe-haut du signal de pression pour produire un signal de pression en courant alternatif, à évaluer le signal de pression en courant alternatif pour détecter une perte de niveau ou effacement dans les composantes de signal imputable à la fréquence cardiaque du patient et à produire une alarme si une chute ou perte de niveau est détectée.

3. Appareil selon la revendication 1, dans lequel les moyens destinés à évaluer le signal de pression en courant alternatif comprennent un détecteur de niveau (179) avec hystérésis, disposé de façon à recevoir le signal filtré provenant du filtre passe-haut (177) et à convertir ce signal en un signal de séquence de pulsations, un discriminateur de fréquences (181) disposé de façon à recevoir le signal de séquence de pulsations et pour produire un signal de sortie ayant un niveau proportionnel à la fréquence du signal de séquence de pulsations, et un comparateur (183) ayant une entrée reliée à la sortie du discriminateur de fréquences et une autre entrée reliée à une référence de tension (191).

Fig. 1

Fig.2.

ARTERIAL INFILTRATION DETECTOR II-35

PRESSURE SIGNAL — 27 → [0.1 Hz HIGH-PASS FILTER] (177) — 183 → [LEVEL DETECTOR W/HYSTERESIS] (179) — 185 → [FREQUENCY DISCRIMINATOR] (181) — 187 → [COMPARATOR] (183) — 193 → [AND] (195) — 197 → [LATCH] (199) — 157 → ALARM SIGNAL

189 + input to comparator from +V / resistor to ground (191)

ARTERIAL ENABLE SIGNAL — 45 → AND gate (195)

8